(19) Europäisches Patentamt
European Patent Office
Office européen des brevets

(11) **EP 3 280 319 B1**

(12) **EUROPEAN PATENT SPECIFICATION**

(45) Date of publication and mention
of the grant of the patent:
**25.09.2024 Bulletin 2024/39**

(21) Application number: **16718911.7**

(22) Date of filing: **08.04.2016**

(51) International Patent Classification (IPC):
*A61B 5/00* (2006.01)        *A61B 5/0205* (2006.01)
*A61B 5/091* (2006.01)       *A61B 5/08* (2006.01)
*A61B 5/1455* (2006.01)      *A61B 5/0245* (2006.01)
*A61B 5/024* (2006.01)

(52) Cooperative Patent Classification (CPC):
**A61B 5/7275; A61B 5/0205; A61B 5/14551;**
**A61B 5/349; A61B 5/7267; G16H 10/60;**
**G16H 40/63; G16H 50/20; G16H 50/30;**
A61B 5/02405; A61B 5/02416; A61B 5/0245;
A61B 5/0816; A61B 5/082; A61B 5/091

(86) International application number:
**PCT/IB2016/051997**

(87) International publication number:
**WO 2016/162838 (13.10.2016 Gazette 2016/41)**

(54) **CARDIOVASCULAR DETERIORATION WARNING SCORE**

WARNWERT FÜR KARDIOVASKULÄRE VERSCHLECHTERUNG

SCORE D'AVERTISSEMENT DE DÉTÉRIORATION CARDIOVASCULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.04.2015 US 201562144372 P**

(43) Date of publication of application:
**14.02.2018 Bulletin 2018/07**

(73) Proprietor: **Koninklijke Philips N.V.
5656 AG Eindhoven (NL)**

(72) Inventors:
• **CHBAT, Nicolas Wadih
5656 AE Eindhoven (NL)**
• **AARTS, Ronaldus Maria
5656 AE Eindhoven (NL)**
• **ZHOU, Sophia Huai
5656 AE Eindhoven (NL)**

(74) Representative: **Philips Intellectual Property &
Standards
High Tech Campus 52
5656 AG Eindhoven (NL)**

(56) References cited:
EP-A2- 1 102 197        WO-A1-2010/053743
WO-A1-2014/042942       WO-A2-2009/005734
US-A1- 2007 191 697     US-A1- 2008 004 904
US-A1- 2008 157 980     US-A1- 2009 093 686
US-A1- 2014 257 122

EP 3 280 319 B1

**Description**

FIELD

The following relates generally to the cardiac care arts, medical emergency response arts, and so forth.

BACKGROUND

Numerous clinical scenarios may arise which might, or might not, be indicative of cardiac deterioration, such as a patient having one or more of the symptoms: feeling dizzy; physical weakness; rapid or irregular heartbeats; shortness of breath; discomfort in the chest; or so forth. Such symptoms are common cardiovascular disease symptoms of patients with potential risks for cardiac arrest, or acute heart attack, or acute heart failure when the symptoms are severe, or for irregular heart rhythm, or coronary artery disease when the symptoms are less severe. Typical situations in which cardiac deterioration is more likely to be present include patients being transported by an ambulance, or admitted to an emergency department of a hospital, or a hospitalized patient after a knee replacement surgery or other surgical or other stressful medical procedure.

Early detection and diagnosis of cardiac deterioration has a significant impact on the ultimate success or failure of cardiac care. Cardiac deterioration mechanisms directly associated with the cardiac muscle include, for example: myocardial ischemia (a reduction in blood flow/oxygenation of the heart), left ventricular hypertrophy (muscle buildup in the left ventricular wall, usually resulting from chronic excessive cardiac effort due to high blood pressure or another condition), systolic heart failure (deterioration in ventricular performance during systolic pumping action, usually correlating with low ejection fraction), and diastolic heart failure (deterioration in ventricular performance during diastole relaxation, usually correlating with low stroke volume). Other cardiac deterioration mechanisms relate to the vasculature servicing the heart, such as valve degradation, or plaque build-up which can lead to stenosis. The appropriate treatment depends upon which of these various cardiac deterioration mechanisms (or combination of mechanisms) is present. Many of these cardiac deterioration mechanisms, if left untreated, can lead to acute debilitating or life-threatening medical events such as cardiac arrest, acute heart attack, acute heart failure, irregular heart rhythm, coronary artery disease, or the like.

Numerous specialized medical tests have been developed to diagnose cardiac deterioration. In practice, however, these are often not ordered for a given patient until the cardiac deterioration has reached an advanced state and has become manifestly symptomatic. Moreover, interpretation of various cardiac tests is difficult, and in the early stages of cardiac deterioration the physician seeing the patient is often not a trained cardiologist but rather a general practice (GP) physician and/or a physician specializing in some other area.

US 2014/0257122 discloses a system for determining a risk score for triage. US 2008/0004904 discloses a medical system that includes a network and one or more medical data collection appliances coupled to the network. US 2008/0157980 discloses a system for predicting heart failure decompensation.

The following discloses a new and improved systems and methods that address the above referenced issues, and others.

SUMMARY

The invention is defined by the independent claim. Dependent claims define advantageous embodiments.

In one disclosed aspect, a patient monitor is disclosed, comprising a display component, a plurality of sensors reading vital signs of a human subject including at one cardiovascular parameter and at least one respiratory parameter, and a microprocessor or microcontroller programmed to perform a cardiovascular early warning scoring (cEWS) method. The cEWS method includes the operations of: (i) classifying the human subject using a plurality of cardiovascular deterioration classifiers each trained to classify the human subject respective to a different type of cardiovascular deterioration to generate cardiovascular early warning scores for the different types of cardiovascular deterioration, the plurality of cardiovascular deterioration classifiers operating on a set of inputs characterizing the human subject including the at least one cardiovascular parameter and the at least one respiratory parameter read by the plurality of sensors; and (ii) outputting the cardiovascular early warning scores for the different types of cardiovascular deterioration on the display component of the patient monitor. The set of inputs may include the at least one cardiovascular parameter read by electrocardiograph electrodes and the at least one respiratory parameter comprising tidal volume read by an airflow sensor.

One advantage resides in facilitating early diagnosis of cardiovascular deterioration, and facilitating early identification of the type of cardiovascular deterioration.

Another advantage resides in providing early diagnosis of myocardial ischemia.

Another advantage resides in providing the foregoing while leveraging and providing the context of a rules-based diagnosis that is heuristic in nature.

Another advantage resides in synergistically combining multiple automated pathways to provide more accurate diagnosis of cardiovascular deterioration.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

FIGURE 1 diagrammatically illustrates a medical monitoring system including a cardiovascular deterioration early warning system.

FIGURE 2 shows resting heart rate tables for men and women.

FIGURE 3 plots maximum, average, and minimum values for systolic and diastolic blood pressure as a function of age.

FIGURE 4 diagrammatically illustrates a combinational variant of the cardiovascular deterioration early warning system of FIGURE 1, for the illustrative example of providing early warning of myocardial ischemia.

## DETAILED DESCRIPTION

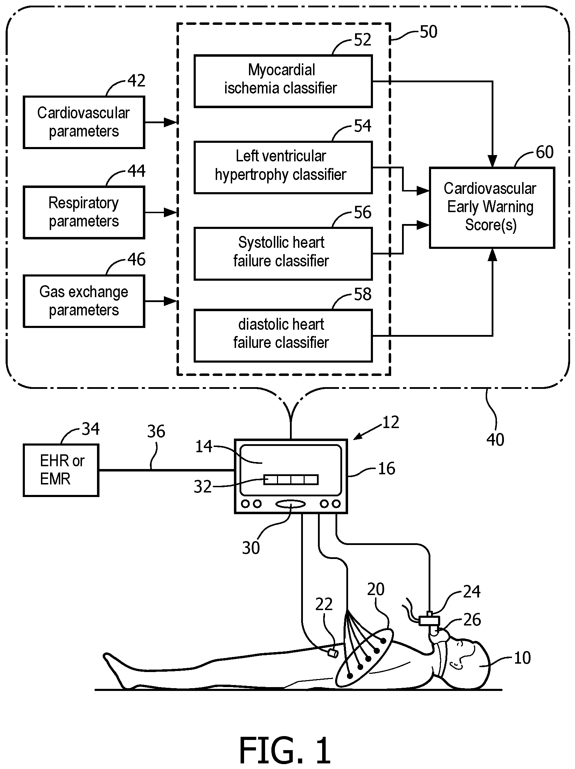

With reference to FIGURE 1, a human subject 10 (such as an in-patient admitted to a hospital, or a nursing home resident, or an outpatient or so forth) is monitored by a patient monitor 12 comprising a display device or component 14, a microprocessor or microcontroller (not shown, but typically housed in a monitor housing 16), and a plurality of physiological (i.e. vital sign) sensors 20, 22, 24 such as an illustrative set of electrocardiograph electrodes 20, a pulse oximeter sensor 22 (not connected as illustrated but typically clipped onto a finger or earlobe of the subject 10), and an airflow sensor 24. In illustrative FIGURE 1 the airflow sensor 24 is installed in a full-face mask 26 used for mechanical ventilation, sleep apnea treatment, general respiratory monitoring or the like. In other embodiments the airflow sensor 24 may be installed in conjunction with a nasal mask or nasal cannula or the like. In general, the patient monitor 12 may be in one of a number of typical settings, such as being installed in a patient room at a hospital (if the subject 10 is an in-patient, could be a specialized hospital area such as the surgical floor, post-anesthesia care unit, intensive care unit, or so forth), or at a nursing home room (if the subject 10 is a nursing home resident), in an ambulance or other emergency response system (EMS) vehicle (if the subject 10 is the subject of an EMS call), or so forth,

The sensors 20, 22, 24 acquire vital sign data in real-time (i.e. continuously, or by sampling with a relatively fast sampling rate) with the vital sign data being optionally processed by algorithms running on the microprocessor or microcontroller of the patient monitor 12. For example, the patient monitor 12 may execute algorithms to generate ECG lead traces from measured voltages of the ECG electrodes 20 and to extract information from the ECG lead traces such as heart rate and presence/absence of associated rate abnormalities (e.g. tachycardia, bradycardia, may use age- and/or gender-specific limits), heart rate variability, QT interval, presence/absence of various arrhythmias such as atrial (AFib), supraventricular tachycardia (SVT), increased QT interval (long QTc), or so forth. The patient monitor 12 may execute algorithms to process the airflow data acquired by the airflow sensor 24 to extract information such as respiratory rate and tidal volume. As another example, the patient monitor 12 may execute algorithms to process a peripheral plethysmograph waveform acquired by the pulse oximeter to derive saturation of peripheral oxygen ($SpO_2$) and heart rate data.

The vital sign sensors 20, 22, 24 are merely illustrative, and additional or other vital sign sensors are contemplated, such as a blood pressure cuff, sphygmomanometer, or other blood pressure sensor or sensors. The patient monitor 12 may process blood pressure date to extract systolic and diastolic blood pressure, with high- or low-blood pressure limits defined that may again be age- and/or gender-specific. Furthermore, if the illustrative mask 26 is part of a mechanical ventilation system (that is, the patient 10 is mechanically ventilated) then other ventilator data additional to the previously mentioned respiration rate and tidal volume may be available and suitably input to the patient monitor 12.

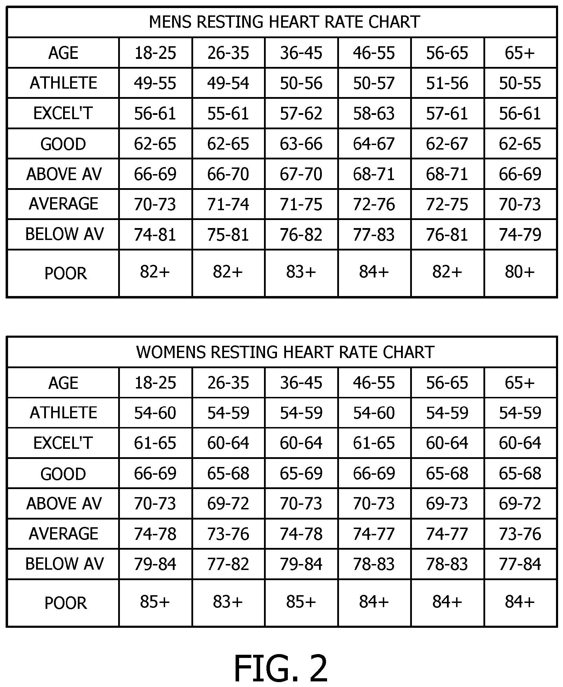

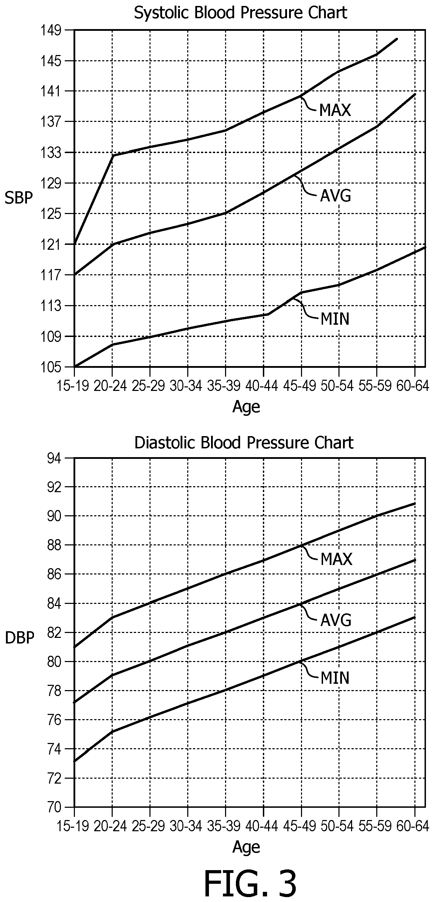

With brief reference to FIGURES 2 and 3, the impact of demographic data on vital signs is illustrated. FIGURE 2 shows resting heart rate tables for men and women, further categorized by age and fitness level. FIGURE 3 plots systolic and diastolic blood pressure (maximum, average, and minimum values) as a function of age.

With reference back to FIGURE 1, the patient monitor 12 is also capable of receiving and storing patient physiological data acquired by laboratory tests or the like. For example, blood gas analysis test results may be received, providing information such as partial pressure of oxygen ($PaO_2$) and/or partial pressure of carbon dioxide ($PaCO_2$). Another contemplated laboratory result is troponin level in the blood. Such data may be entered into the patient monitor 12 manually, e.g. using a physical keypad 30 or soft keys 32 on the display 14 (the soft keys 32 are implementable if the display 14 is a touch-sensitive display). In one embodiment, respiratory rate is determined manually, for example by a nurse visually assessing respiratory rate, and the manually determined respiratory rate is entered into the patient monitor 12 using the input(s) 30, 32. Other patient data such as demographic data (e.g. age, gender), medical history, perioperative status data, and the like may be similarly provided to the patient monitor 12. Rather than being manually entered via a user interface 30, 32 of the patient monitor 12, such information may be input to the patient monitor 12 from an Electronic Health Record (EHR), Electronic Medical Record (EMR), or the like 34 via a hospital data network or other electronic data network 36 if the patient monitor 12 is connected with such patient records storage and communication infrastructure 34, 36.

The illustrative patient monitor 12 further includes a cardiovascular deterioration early warning scoring (cEWS) (sub-)system 40 that is diagrammatically depicted in FIGURE 1 by functional blocks suitably executed by the microprocessor or microcontroller of the patient monitor **12**. The illustrative cEWS system **40** receives as inputs patient values (i.e. vital sign readings or sensor values, possibly processed) for cardiovascular parameters **42** such as ECG-derived data, heart rate from the pulse oximeter **22**), blood pressure data, or so forth. Such parameters (or at least a sub-set of them) are readily recognized as being pertinent to assessing cardiovascular deterioration.

Additionally, the cEWS system **40** receives at least one respiratory parameter **44**, such as respiratory rate, tidal volume, or so forth. The illustrative cEWS system **40** also receives at least one gas exchange parameter **46**, such as $SpO_2$ from the pulse oximeter **22**, or $PaO_2$ and/or $PaCO_2$ values from blood gas analysis, or so forth. It is recognized herein that these additional parameters **44**, **46**, although not characterizing the cardiovascular system directly, are of value in assessing cardiovascular deterioration because the respiratory and gas exchange systems characterize the pulmonary system which together with the cardiovascular system forms an integrated cardiopulmonary system.

The cEWS system **40** comprises a plurality of cardiovascular deterioration classifiers (i.e. inference engines) **50**, one for each type of cardiovascular deterioration of interest. The illustrative cEWS system **40** includes a myocardial ischemia classifier **52**, a left ventricular hypertrophy classifier **54**, a systolic heart failure classifier **56**, and a diastolic heart failure classifier **58**. There are merely illustrative, and classifiers trained to detect other types of cardiovascular deterioration such as cardiac valve deterioration, low cardiac output, cardiac arterial stenosis, or so forth are additionally or alternatively contemplated. Each classifier **52**, **54**, **56**, **58** may, for example, be a neural network, support vector machine, a nonlinear regression model (e.g. logistic or polynomial regression), or other type of classifier. It will be appreciated that the classifiers **52**, **54**, **56**, **58** may in general be of different types.

In general, each classifier **52**, **54**, **56**, **58** is trained using a labeled data set $\{(\bar{x}_i , y_i)\}$, $i = 1, ..., N$ comprising $N$ past (training) patients, with each training patient i being represented by a vector $\bar{x}_i$ of patient data (e.g. vital signs, demographic data, patient history data) and a label $y_i$. The elements of the training patient data vector $\bar{x}_i$ are referred to herein as "features" in accord with common usage in the classifier training arts. The label $y_i$ represents whether the training patient i was diagnosed with the cardiac deterioration for which the classifier is being trained. For example, in training the ischemia classifier **52** the label $y_i$ may be a binary value indicating whether the training patient $i$ was diagnosed with cardiac ischemia. Alternatively, the label may be more informative, e.g. the label $y_i$ for training the ischemia classifier **52** may be integer value in the range between 0 and 5, where a value of 0 indicates the training patient was diagnosed with no detectable cardiac ischemia, a value of 5 indicates the training patient was diagnosed with ischemia of highest severity, and the values 1, .., 4 denote ischemia in the training patient at intermediate severity levels. Continuous outputs are also contemplated, e.g. in a range [0,1] where 0 indicates no detectable ischemia and 1 indicates highest severity ischemia. The classifier is trained to minimize an error metric between its outputs (i.e., "predictions" $\hat{y}_i$) for input training patient data sets $\bar{x}_i$ and the corresponding actual (*a priori* known) labels $y_i$. For example, a simple least squared error of

$$\sum_{i=1}^{N}(\hat{y}_i - y_i)^2$$

the form may be used. The trained classifier outputs predictions y in a format (binary, multi-level, or so forth) which may in general be different for each of the different classifiers **52**, **54**, **56**, **58**.

The trained classifiers **52**, **54**, **56**, **58** are applied to the patient **10**, who is not one of the training patients, and for whom the status of cardiac deterioration (if any) is not known *a priori*. In applying the classifiers **52**, **54**, **56**, **58** to the patient **10**, the patient data **42**, **44**, **46** are formulated in the same manner as the training patient data vectors $\bar{x}_i$. It should be noted that the format and/or content of the vector $\bar{x}_i$ may be different for each different classifier **52**, **54**, **56**, **58**. For example, some training approaches employ a features reduction operation, or some features that are not expected to be relevant to the mode of cardiac deterioration under training may be omitted, so that the vector $\bar{x}_i$ for that classifier is some sub-set of the available patient data **42**, **44**, **46**. The output of each type-specific classifier **52**, **54**, **56**, **58** is a prediction y of whether the subject **10** has that type of cardiac deterioration, or if the output is multi-level or continuous the prediction y encompasses the level of severity of that type of cardiac deterioration in the subject **10**. It should be noted that the predictions y may have a different format from the labels $y_i$ - for example, the labels may be binary values (0=patient not diagnosed with this type of cardiac deterioration; 1=patient was so diagnosed) but the predictions y may be continuous values in the range [0,1]. A continuous-valued prediction in the range [0,1] advantageously may be interpreted as a probability and, for example, written as a percentage in the range 0-100%.

In some embodiments, the prediction outputs may be tied to clinical guidelines used by the ER, EMS, or other medical provider. For example, in an EMS call setting, if the myocardial ischemia score is sufficiently high the output may (in addition to identifying a probable ischemia condition) present the ischemia therapy called for in the clinical guideline for treating ischemia.

It is contemplated that the classifiers **52**, **54**, **56**, **58** may be re-trained occasionally to more accurately reflect current patient demographics.

The use in the cEWS system **40** of the plurality of classifiers **52**, **54**, **56**, **58**, one for each type of cardiac deterioration of interest, recognizes that different types of cardiac deterioration, though somewhat interrelated, have distinct characteristics, so that a single classifier would be unlikely to be effective. The output predictions y of the set of classifiers **52**, **54**, **56**, **58** may be variously combined and/or presented as one or more cardiovascular early warning scores **60**. In one approach, only the highest (i.e. most severe) prediction (score) is presented, and then only if that highest prediction is higher than some threshold. This approach is particularly advantageous in a setting such as an emergency room (ER) or emergency medical service (EMS) call, where medical personnel are dealing with a triage situation and need to be made aware of only the most severe condition. In a variant approach also suitable for triage situations, each classifier score is presented individually but only if its value (i.e. severity) is greater than some (possibly type-specific) threshold. To reduce the information that needs to be processed by emergency medical personnel, it is further contemplated to present the predictions (scores) in some discretized fashion, for example a value of "HIGH" or "MODERATE" depending on the score. Other readily perceived formats are contemplated, such as displaying each prediction as a slider or scale running (for example), with the low end labeled to indicate no likelihood of that type of cardiac deterioration and the high end labeled to indicate a high likelihood of that type of cardiac deterioration. Color coding may also be used, e.g. displaying high scores in red, moderate scores in yellow, and low scores in green. The scores are suitably displayed on the display **14** of the patient monitor **12**, although other outputs are contemplated such as an audible alarm in the case of a very high score. In embodiments suited for non-emergency situations, it is contemplated to present all cEWS values, e.g. as percent probabilities or other numerical values. More generally, the cEWS values can be used for continuous monitoring, for example displayed as a trend line, numeric values updated in real time, or so forth in an ambulance or other mobile emergency response setting, at the hospital room bedside, at a nurses' station, or so forth.

The cEWS system **40** diagrammatically shown in FIGURE 1 is a data-driven system that relies upon empirically trained inference engines **52**, **54**, **56**, **58** to provide predictions (i.e. early warning scores) of various types of cardiac deterioration. The approach provides scores for different types of cardiac deterioration, thus enabling medical personnel without cardiac specialization to make an early assessment of incipient cardiac degradation so that a more detailed cardiac assessment can be performed. It should be noted that the cardiac deterioration scores are not medical diagnoses, but rather early warning indicators which may be considered by a physician, along with other information such as a physical examination, various laboratory test results, and so forth to guide initial triage and/or assist the physician in early detection of cardiovascular deterioration. In general, it is expected that more detailed cardiac assessment will be triggered by the early warning provided by the cEWS system **40** in order to obtain a diagnosis of any cardiac deterioration actually present in the patient **10**.

One possible difficulty with the cEWS system **40** of FIGURE 1 is that, as a purely empirical system, its operation is not as transparent as, for example, heuristic diagnostic rules commonly relied upon by physicians. The empirical approach may also be difficult to correlate with the underlying physiology. This can introduce certain difficulties. The lack of readily apparent correlation with heuristic diagnostic rules and underlying physiology may cause medical personnel to resist relying upon the early warning scores generated by the cEWS system **40**. Also, there may be disadvantages to substituting the cEWS system **40** for existing heuristic diagnostic rules or first-principles physiological analysis. For example, inference engines can suffer from excessive random error if the training data set is too small, or can suffer from systematic error if there are systematic deficiencies in the training data, such as a systematic predisposition to over-diagnose (or under-diagnose) a particular type of cardiac deterioration which is captured in the annotated labels $y_i$ for that type. The empirical training can also capture spurious correlations.

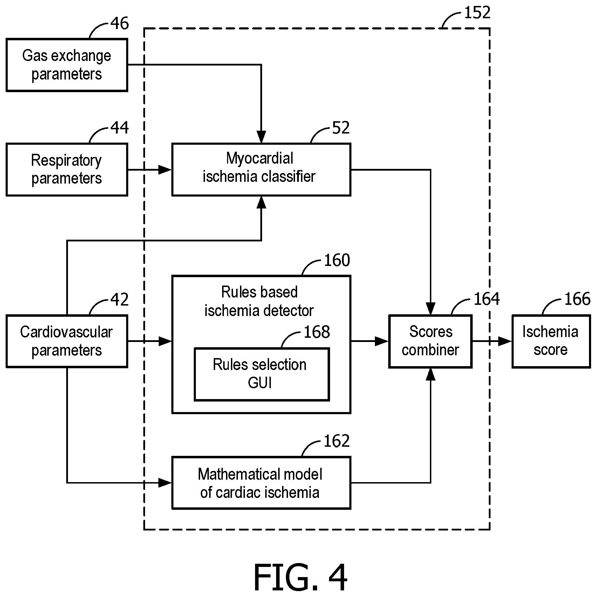

With reference to FIGURE 4, these difficulties are addressed in a variant example of the cardiovascular deterioration type-specific classifiers **52**, **54**, **56**, **58**. In illustrative FIGURE 4, a variant example of **152** the myocardial ischemia classifier **52** of FIGURE 1 is described; however, the extensions to the myocardial ischemia classifier **52** described herein with reference to FIGURE 4 are readily applied to any of the other cardiovascular deterioration type-specific classifiers **54**, **56**, **58**. As seen in FIGURE 4, the variant myocardial ischemia classifier **152** incorporates the myocardial ischemia classifier **52** as a component, and this component (as in FIGURE 1) receives as inputs the cardiovascular parameters **42**, at least one respiratory parameter **44**, and optionally at least one gas exchange parameter **46**. The cardiac ischemia classifier **152** further incorporates two additional cardiac ischemia detection components: a codified rules-based cardiac ischemia detector **160** and a physiological model component that mathematically models the physiology and progression of cardiac ischemia **162**. A scores combiner **164** combines the outputs of the constituent myocardial ischemia detectors **52**, **160**, **162**, for example using a weighted sum of their outputs, to generate an ischemia score **166** that may be output by itself as a myocardial ischemia detector, or may be employed in the cEWS system **40** of FIGURE 1 in place of the output of the empirical myocardial ischemia classifier **52**. (In other words, the modified ischemia detector **152** of FIGURE 4 may be substituted for the ischemia classifier **52** of FIGURE 1).

In the approach of FIGURE 4, the rules-based ischemia detector **160** provides the physician with a familiar component. Various heuristic rules are employed by different cardiologists, different hospitals, or so forth.

The rules used by a particular cardiologist may be a standard set of rules, such as the rules promulgated in the 2013 ACC/AHA guideline on the assessment of cardiovascular risk: a report of the American College of Cardiology/American Heart Association Task Force on Practice Guidelines. http://circ.ahajournals.org/content/early/2013/11/11/01.cir.0000437741.48606.98). On the other hand, a given cardiologist or hospital may employ a variant of the standard rules, or may prefer to employ a standard set of rules promulgated by a different authority. To accommodate such individual and/or institutional preferences, as we as to reassure the cardiologist regarding which rules are being employed, the illustrative rules-based ischemia detector **160** includes a rules selection graphical user interface (GUI) **168** via which the user can select from amongst one, two, three, or more standard rules sets (e.g. the 2013 ACC/AHA 2013 guideline, an earlier edition of the ACC/AHA guideline, and/or a guideline promulgated by another cardiovascular care authority). The GUI **168** is suitably implemented by the microprocessor or microcontroller patient monitor **12** programmed to implement the GUI **168** using the display device or component **14** and the user input device(s) **30**, **32**.

In one illustrative implementation, of the myocardial ischemia detector of FIGURE 4, the set of inputs **42**, **44**, **46** includes troponin levels, blood pressure, disease history, sex, age, and other demographic information like Body Mass Index (BMI) and others and so forth. Optionally, an automated feature selection algorithm employing a regression-type model is employed to identify the most probative features for detecting ischemia, which compares its fitting results with training data. A fit metric such as the correlation coefficient or the coefficient of variation can be used to judge the quality of the fit. The features included in the best fit model will then serve as the features or guides to the three ischemia detector components **52**, **160**, **162** which are described in turn below.

The ischemia classifier **52**, already described with reference to FIGURE 1, is a data-driven component. The data-based algorithm can, for example, be a data mining, machine learning, or statistical correlation type model for ischemia detection. Examples of such algorithms include neural network or logistic regression classifiers.

The rules-based ischemia detector **160** is a codification of the heuristic rules used by the physician in performing ischemia detection. The rules can be codified using a fuzzy inference engine where heuristic rules are translated into mathematical formulation giving crisp features to be selected. Some suitable rules that could be implemented via the rules-based ischemia detector **160** include the aforementioned 2013 ACC/AHA guideline, and/or the AHA/ACCF/HRS Recommendations for the Standardization and Interpretation of the Electrocardiogram Part VI: Acute Ischemia/Infarction (Circulation. 2009;119:e262-e27). In typical guidelines for cardiovascular deterioration, the guidelines rely upon cardiovascular parameters, but typically not on respiratory or gas exchange parameters. Accordingly, the illustrative rules-based ischemia detector **160** receives as input the cardiovascular parameters **42** but not the at least one respiratory parameter **44** and not the at least one gas exchange parameter **46**. (However, it is also contemplated for the rules-based ischemia detector to employ rules additionally operating on respiratory and/or gas exchange parameter(s)).

The physiological model component **162** comprises static (algebraic) and/or dynamic (differential) equations that articulate ischemic deterioration of the myocardium. This knowledge is obtained from the pathophysiological understanding of myocardial ischemia. The knowledge is then expressed mathematically. Typical physiological models of cardiovascular deterioration rely upon cardiovascular parameters, but typically not on respiratory or gas exchange parameters. Accordingly, the illustrative physiological model-based detector **162** receives as input the cardiovascular parameters **42** but not the at least one respiratory parameter **44** and not the at least one gas exchange parameter **46**. (However, it is also contemplated for the physiological model-based detector to employ rules additionally operating on respiratory and/or gas exchange parameter(s)).

The outputs of the three detectors **52**, **160**, **162** are updated at each instance that a patient data record is presented/updated. Each detector **52**, **160**, **162** outputs an assessment (i.e. score) estimating the onset of ischemia. The three outputs are then aggregated via the scores combiner **164** to generate the ischemia score value **166**. In some embodiments, the scores combiner **164** normalizes the input and output to produce the ischemia score **166** in the range [0%,100%] where a score of 0% indicates lowest estimated likelihood/severity of cardiac ischemia, while 100% represents highest likelihood/severity of cardiac ischemia. The ischemia score **166** may, in general, evolve over time as parameters such as heart rate, respiration rate, tidal volume, blood pressure, and so forth are updated by readings of the sensors **20**, **22**, **24** and/or as other inputs such as blood gas analysis results are input to the system.

The weights for the scores output by the respective detectors **52**, **160**, **162** are suitably determined (or fine-tuned) during the training phase by optimizing the fit between the output **166** and the annotated labels $y_i$ pertaining to cardiac ischemia. The scores combiner **164** may employ a simple weighted average or weighted sum of the outputs of the constituent ischemia detectors **52**, **160**, **162**. In other embodiments, the scores combiner **164** performs the weighted aggregation using a more sophisticated technique such as Linear Discriminator Analysis (LDA) to provide the single value **166** of ischemia detection.

As already mentioned, the output **166** may be employed in the context of the cEWS system **40** of FIGURE 1, substituting for the output of the ischemia classifier **52** in this cEWS system **40**. In other embodiments, the system of FIGURE 4 operates as a stand-alone myocardial ischemia detector, and the level of detected ischemia severity can be displayed as a binary value (e.g. indicating possible cardiac ischemia if the aggregate score **166** is above a certain threshold), or quantized to one of more than two levels (multi-level discretized output) for example represented as a "traffic light" with green color indicating low ischemia likelihood/severity, yellow indicating moderate ischemia likelihood/severity, and red indicating high ischemia likelihood/severity. Additionally or alternatively, the ischemia score **166** may be presented as a numeric value (updated in real time), or as a trend line.

While the aggregate score **166** is expected to be more accurate than the individual outputs of the respective ischemia detector components **52**, **160**, **162**, it is also contemplated to display the outputs of the individual ischemia detector components **52**, **160**, **162**, for example using one of the above-mentioned binary, color coded, numeric, and/or trend line representations.

While illustrative FIGURE 4 combines the empirical ischemia classifier **52**, rules-based ischemia detector **160**, and physiological model-based detector **162**, it is alternatively contemplated to include only two of these components **52**, **160**, **162**. For example, the physiological model-based detector **162**, is optionally omitted. Furthermore, as already mentioned, it will be appreciated any of the other illustrative cardiovascular deterioration modality classifiers **54**, **56**, **58** may be similarly modified to further incorporate a rule-based detector component and/or a physiological model-based detector component.

It will be appreciated that the disclosed cardiovascular deterioration early warning system cEWS system **40**, and/or the constituent classifiers **52**, **54**, **56**, **58**, **152** or stand-alone modality detector **152**, may also be embodied as a non-transitory storage medium storing instructions readable and executable by the microprocessor or microcontroller of the patient monitor **12**, or by another electronic data processing device, to perform the disclosed cardiovascular deterioration detection operations. Such a non-transitory storage medium may, by way of illustration, include: a hard disk drive or other magnetic storage medium; an optical disk or other optical storage medium; a read-only memory (ROM), electronically programmable read-only-memory (PROM), flash memory or other electronic storage medium; various combinations thereof; and so forth.

The invention has been described with reference to the preferred embodiments. Modifications and alterations may occur to others upon reading and understanding the preceding detailed description. It is intended that the invention be construed as including all such modifications and alterations insofar as they come within the scope of the appended claims.

**Claims**

1. A patient monitor comprising:

    a display component (14);
    a plurality of sensors (20, 22, 24) configured for reading vital signs of a human subject including at least one cardiovascular parameter and at least one respiratory parameter; and
    a microprocessor or microcontroller programmed to perform a cardiovascular early warning scoring (cEWS) method including the operations of:

        (i) classifying the human subject using a plurality of cardiovascular deterioration classifiers (52, 152, 54, 56, 58) each trained to classify the human subject respective to a different type of cardiovascular deterioration to generate cardiovascular early warning scores for the different types of cardiovascular deterioration, the plurality of cardiovascular deterioration classifiers operating on a set of inputs characterizing the human subject including the at least one cardiovascular parameter (42) and the at least one respiratory parameter (44) read by the plurality of sensors, and
        (ii) outputting the cardiovascular early warning scores for the different types of cardiovascular deterioration on the display component of the patient monitor.

2. The patient monitor of claim 1 wherein the plurality of cardiovascular deterioration classifiers operate on the set of inputs including the at least one cardiovascular parameter (42) read by electrocardiograph electrodes (20) and the at least one respiratory parameter (44) comprising tidal volume read by an airflow sensor (24).

3. The patient monitor of claim 2 wherein the at least one respiratory parameter (44) further includes respiration rate.

4. The patient monitor of any one of claims 1-3 wherein the set of inputs characterizing the human subject further include at least one gas exchange parameter (46) read by the plurality of sensors (20, 22, 24).

5. The patient monitor of claim 4 wherein the plurality of cardiovascular deterioration classifiers operate on the set of inputs including the at least gas exchange parameter (46) comprising saturation of peripheral oxygen (SpO$_2$) read by a pulse oximeter sensor (22).

6. The patient monitor of any one of claims 1-5 wherein the plurality of cardiovascular deterioration classifiers (52, 152, 54, 56, 58) operate on the set of inputs characterizing the human subject further including blood gas analysis test results including at least one of partial pressure of oxygen (PaO$_2$) and partial pressure of carbon dioxide (PaCO$_2$),
wherein the blood gas analysis test results are input to the patient monitor by one of a user input device (30, 32) and reading an Electronic Health Record (EHR) or Electronic Medical Record (EMR) (34) via an electronic data network (36).

7. The patient monitor of claim 6 wherein the plurality of cardiovascular deterioration classifiers (52, 152, 54, 56, 58) operate on the set of inputs including said blood gas analysis test results further including troponin level in the blood.

8. The patient monitor of any one of claims 1-7 wherein the plurality of cardiovascular deterioration classifiers (52, 152, 54, 56, 58) include at least two classifiers of the group of cardiovascular deterioration classifiers consisting of a myocardial ischemia classifier (52, 152), a left ventricular hypertrophy classifier (54), a systolic heart failure classifier (56), and a diastolic heart failure classifier (58).

9. The patient monitor of any one of claims 1-8 wherein the plurality of cardiovascular deterioration classifiers includes a first cardiovascular deterioration classifier (152) classifying the human subject respective to a first type of cardiovascular deterioration to generate a cardiovascular early warning score for the first type of cardiovascular deterioration, wherein the first cardiovascular deterioration classifier comprises:

an empirical classifier (52) trained using labeled training data to generate an empirical score for the first type of cardiovascular deterioration;
a rules-based cardiovascular deterioration detector (160) applying a set of rules to generate a rules-based score for the first type of cardiovascular deterioration; and
a scores combiner generating a weighted combination of scores for the first type of cardiovascular deterioration including at least the empirical score and the rules-based score.

10. The patient monitor of claim 9 wherein the first cardiovascular deterioration classifier further comprises:

a physiological model-based detector (162) modeling the first type of cardiovascular deterioration using algebraic or differential equations to generate a model-based score for the first type of cardiovascular deterioration;
wherein the scores combiner generates the weighted combination of scores for the first type of cardiovascular deterioration including the empirical score, the rules-based score, and the model-based score.

## Patentansprüche

1. Patientenmonitor, umfassend:

eine Anzeigekomponente (14);
eine Vielzahl von Sensoren (20, 22, 24), die zum Auslesen von Vitalzeichen eines menschlichen Subjekts konfiguriert sind, einschließlich mindestens eines kardiovaskulären Parameters und mindestens eines Atmungsparameters; und
einen Mikroprozessor oder Mikrocontroller, der zum Durchführen eines Verfahrens zur kardiovaskulären Frühwarnbewertung (cEWS) programmiert ist, das die folgenden Vorgänge einschließt:

(i) Klassifizieren des menschlichen Subjekts unter Verwendung einer Vielzahl von Klassifikatoren (52, 152, 54, 56, 58) für eine kardiovaskuläre Verschlechterung, die jeweils trainiert sind, um das menschliche Subjekt in Bezug auf eine unterschiedliche Art der kardiovaskulären Verschlechterung zu klassifizieren, um kardiovaskuläre Frühwarnwerte für die unterschiedlichen Arten der kardiovaskulären Verschlechterung zu erzeugen, wobei die Vielzahl von Klassifikatoren für eine kardiovaskuläre Verschlechterung mit einem Satz von Eingaben arbeitet, die das menschliche Subjekt charakterisieren, einschließlich des mindestens einen kardiovaskulären Parameters (42) und des mindestens einen Atmungsparameters (44), die von der Vielzahl von Sensoren ausgelesen werden, und
(ii) Ausgeben der kardiovaskulären Frühwarnwerte für die verschiedenen Arten der kardiovaskulären Verschlechterung auf der Anzeigekomponente des Patientenmonitors.

2. Patientenmonitor nach Anspruch 1, wobei die Vielzahl von Klassifikatoren für eine kardiovaskuläre Verschlechterung mit dem Satz von Eingaben arbeitet, der den mindestens einen kardiovaskulären Parameter (42), der von Elektrokardiographieelektroden (20) ausgelesen wird, und den mindestens einen Atmungsparameter (44) einschließt, der das von einem Luftstromsensor (24) ausgelesene Tidalvolumen umfasst.

**3.** Patientenmonitor nach Anspruch 2, wobei der mindestens eine Atmungsparameter (44) weiter die Atemfrequenz einschließt.

**4.** Patientenmonitor nach einem der Ansprüche 1-3, wobei der Satz von Eingaben, die das menschliche Subjekt charakterisieren, weiter mindestens einen Gasaustauschparameter (46) einschließt, der von der Vielzahl von Sensoren (20, 22, 24) ausgelesen wird.

**5.** Patientenmonitor nach Anspruch 4, wobei die Vielzahl von Klassifikatoren für eine kardiovaskuläre Verschlechterung mit dem Satz von Eingaben arbeitet, der mindestens den Gasaustauschparameter (46) einschließt, der die von einem Pulsoximetersensor (22) ausgelesene Sättigung des peripheren Sauerstoffs (SpOj) umfasst.

**6.** Patientenmonitor nach einem der Ansprüche 1-5, wobei die Vielzahl von Klassifikatoren (52, 152, 54, 56, 58) für eine kardiovaskuläre Verschlechterung mit dem Satz von Eingaben arbeitet, die das menschliche Subjekt charakterisieren und weiter Testergebnisse der Blutgasanalyse einschließen, die mindestens einen Partialdruck von Sauerstoff (PaO$_2$) und einen Partialdruck von Kohlendioxid (PaCO$_2$) einschließen,
wobei die Testergebnisse der Blutgasanalyse entweder durch eine Benutzereingabevorrichtung (30, 32) oder durch Auslesen einer elektronischen Gesundheitsakte (EHR) oder einer elektronischen Patientenakte (EMR) (34) über ein elektronisches Datennetz (36) in den Patientenmonitor eingegeben werden.

**7.** Patientenmonitor nach Anspruch 6, wobei die Vielzahl von Klassifikatoren (52, 152, 54, 56, 58) für eine kardiovaskuläre Verschlechterung mit dem Satz von Eingaben arbeitet, der die Testergebnisse der Blutgasanalyse einschließt und weiter den Troponinspiegel im Blut einschließt.

**8.** Patientenmonitor nach einem der Ansprüche 1-7, wobei die Vielzahl von Klassifikatoren (52, 152, 54, 56, 58) für eine kardiovaskuläre Verschlechterung mindestens zwei Klassifikatoren aus der Gruppe der Klassifikatoren für eine kardiovaskuläre Verschlechterung einschließt, die aus einem Klassifikator (52, 152) für myokardiale Ischämie, einem Klassifikator (54) für linksventrikuläre Hypertrophie, einem Klassifikator (56) für systolische Herzinsuffizienz und einem Klassifikator (58) für diastolische Herzinsuffizienz besteht.

**9.** Patientenmonitor nach einem der Ansprüche 1-8, wobei die Vielzahl von Klassifikatoren für eine kardiovaskuläre Verschlechterung einen ersten Klassifikator (152) für eine kardiovaskuläre Verschlechterung einschließt, der das menschliche Subjekt in Bezug auf eine erste Art der kardiovaskulären Verschlechterung klassifiziert, um einen kardiovaskulären Frühwarnwert für die erste Art der kardiovaskulären Verschlechterung zu erzeugen, wobei der erste Klassifikator für eine kardiovaskuläre Verschlechterung umfasst:

einen empirischen Klassifikator (52), der mit bezeichneten Trainingsdaten trainiert wird, um eine empirische Bewertung für die erste Art der kardiovaskulären Verschlechterung zu erzeugen;
einen regelbasierten Detektor (160) für kardiovaskuläre Verschlechterung, der einen Satz von Regeln anwendet, um eine regelbasierte Bewertung für die erste Art der kardiovaskulären Verschlechterung zu erzeugen; und
einen Bewertungskombinierer, der eine gewichtete Kombination von Bewertungen für die erste Art der kardiovaskulären Verschlechterung erzeugt, die mindestens die empirischen Bewertung und die regelbasierte Bewertung einschließt.

**10.** Patientenmonitor nach Anspruch 9, wobei der erste Klassifikator für die kardiovaskuläre Verschlechterung weiter umfasst:

einen physiologischen modellbasierten Detektor (162), der die erste Art einer kardiovaskulären Verschlechterung unter Verwendung algebraischer oder differentieller Gleichungen modelliert, um eine modellbasierte Bewertung für die erste Art der kardiovaskulären Verschlechterung zu erzeugen;
wobei der Bewertungskombinierer die gewichtete Kombination von Bewertungen für die erste Art der kardiovaskulären Verschlechterung erzeugt, die die empirische Bewertung, die regelbasierte Bewertung und die modellbasierte Bewertung einschließt.

**Revendications**

**1.** Moniteur de patient comprenant :

un composant d'affichage (14) ;
une pluralité de capteurs (20, 22, 24) configurés pour lire les signes vitaux d'un sujet humain, incluant au moins un paramètre cardiovasculaire et au moins un paramètre respiratoire ; et
un microprocesseur ou un micro-dispositif de commande programmé pour exécuter un procédé d'évaluation avertissement cardiovasculaire précoce (cEWS) incluant les opérations consistant à :

(i) classer le sujet humain à l'aide d'une pluralité de classificateurs de détérioration cardiovasculaire (52, 152, 54, 56, 58), chacun étant entraîné pour classer le sujet humain en fonction d'un type différent de détérioration cardiovasculaire afin de générer des scores d'avertissement cardiovasculaire précoce pour les différents types de détérioration cardiovasculaire, la pluralité de classificateurs de détérioration cardiovasculaire fonctionnant sur un ensemble d'entrées caractérisant le sujet humain incluant le au moins un paramètre cardiovasculaire (42) et le au moins un paramètre respiratoire (44) lus par la pluralité de capteurs, et
(ii) délivrer en sortie les scores d'avertissement cardiovasculaire précoce pour les différents types de détérioration cardiovasculaire sur le composant d'affichage du moniteur de patient.

2. Moniteur de patient selon la revendication 1 dans lequel la pluralité de classificateurs de détérioration cardiovasculaire fonctionnent sur l'ensemble d'entrées incluant le au moins un paramètre cardiovasculaire (42) lu par des électrodes d'électrocardiographe (20) et le au moins un paramètre respiratoire (44) comprenant le volume courant lu par un capteur d'écoulement d'air (24).

3. Moniteur de patient selon la revendication 2 dans lequel le au moins un paramètre respiratoire (44) inclut en outre la fréquence respiratoire.

4. Moniteur de patient selon l'une quelconque des revendications 1-3 dans lequel l'ensemble d'entrées caractérisant le sujet humain inclut en outre au moins un paramètre d'échange gazeux (46) lu par la pluralité de capteurs (20, 22, 24).

5. Moniteur de patient selon la revendication 4 dans lequel la pluralité de classificateurs de détérioration cardiovasculaire fonctionnent sur l'ensemble d'entrées incluant le au moins paramètre d'échange gazeux (46) comprenant la saturation en oxygène périphérique (SpO$_2$) lue par un capteur d'oxymètre de pouls (22).

6. Moniteur de patient selon l'une quelconque des revendications 1-5 dans lequel la pluralité de classificateurs de détérioration cardiovasculaire (52, 152, 54, 56, 58) fonctionnent sur l'ensemble d'entrées caractérisant le sujet humain incluant en outre des résultats de test d'analyse des gaz du sang incluant au moins l'une d'une pression partielle d'oxygène (PaO$_2$) et d'une pression partielle de dioxyde de car-

bone (PaCO$_2$),
dans lequel les résultats de test d'analyse des gaz du sang sont entrés dans le moniteur de patient par l'un d'un dispositif d'entrée utilisateur (30, 32) et d'une lecture d'un dossier de santé électronique (DSE) ou d'un dossier médical électronique (DME) (34) via un réseau de données électroniques (36).

7. Moniteur de patient selon la revendication 6 dans lequel la pluralité de classificateurs de détérioration cardiovasculaire (52, 152, 54, 56, 58) fonctionnent sur l'ensemble d'entrées incluant lesdits résultats de test d'analyse des gaz du sang incluant en outre le niveau de troponine dans le sang.

8. Moniteur de patient selon l'une quelconque des revendications 1-7, dans lequel la pluralité de classificateurs de détérioration cardiovasculaire (52, 152, 54, 56, 58) incluent au moins deux classificateurs du groupe de classificateurs de détérioration cardiovasculaire consistant en un classificateur d'ischémie myocardique (52, 152), un classificateur d'hypertrophie ventriculaire gauche (54), un classificateur d'insuffisance cardiaque systolique (56) et un classificateur d'insuffisance cardiaque diastolique (58).

9. Moniteur de patient selon l'une quelconque des revendications 1-8 dans lequel la pluralité de classificateurs de détérioration cardiovasculaire inclut un premier classificateur de détérioration cardiovasculaire (152) classant le sujet humain selon un premier type de détérioration cardiovasculaire pour générer un score d'avertissement cardiovasculaire précoce pour le premier type de détérioration cardiovasculaire, dans lequel le premier classificateur de détérioration cardiovasculaire comprend :

un classificateur empirique (52) entraîné à l'aide de données d'entraînement étiquetées pour générer un score empirique pour le premier type de détérioration cardiovasculaire ;
un détecteur de détérioration cardiovasculaire à base de règles (160) appliquant un ensemble de règles pour générer un score à base de règles pour le premier type de détérioration cardiovasculaire ; et
un combineur de scores générant une combinaison pondérée de scores pour le premier type de détérioration cardiovasculaire incluant au moins le score empirique et le score à base de règles.

10. Moniteur de patient selon la revendication 9 dans lequel le premier classificateur de détérioration cardiovasculaire comprend en outre :

un détecteur à base de modèle physiologique (162) modélisant le premier type de détérioration cardiovasculaire à l'aide d'équations algébriques ou différentielles pour générer un score à base de modèle pour le premier type de détérioration cardiovasculaire ;

dans lequel le combineur de scores génère la combinaison pondérée de scores pour le premier type de détérioration cardiovasculaire incluant le score empirique, le score à base de règles et le score à base de modèle.

FIG. 1

| MENS RESTING HEART RATE CHART | | | | | | |
|---|---|---|---|---|---|---|
| AGE | 18-25 | 26-35 | 36-45 | 46-55 | 56-65 | 65+ |
| ATHLETE | 49-55 | 49-54 | 50-56 | 50-57 | 51-56 | 50-55 |
| EXCEL'T | 56-61 | 55-61 | 57-62 | 58-63 | 57-61 | 56-61 |
| GOOD | 62-65 | 62-65 | 63-66 | 64-67 | 62-67 | 62-65 |
| ABOVE AV | 66-69 | 66-70 | 67-70 | 68-71 | 68-71 | 66-69 |
| AVERAGE | 70-73 | 71-74 | 71-75 | 72-76 | 72-75 | 70-73 |
| BELOW AV | 74-81 | 75-81 | 76-82 | 77-83 | 76-81 | 74-79 |
| POOR | 82+ | 82+ | 83+ | 84+ | 82+ | 80+ |

| WOMENS RESTING HEART RATE CHART | | | | | | |
|---|---|---|---|---|---|---|
| AGE | 18-25 | 26-35 | 36-45 | 46-55 | 56-65 | 65+ |
| ATHLETE | 54-60 | 54-59 | 54-59 | 54-60 | 54-59 | 54-59 |
| EXCEL'T | 61-65 | 60-64 | 60-64 | 61-65 | 60-64 | 60-64 |
| GOOD | 66-69 | 65-68 | 65-69 | 66-69 | 65-68 | 65-68 |
| ABOVE AV | 70-73 | 69-72 | 70-73 | 70-73 | 69-73 | 69-72 |
| AVERAGE | 74-78 | 73-76 | 74-78 | 74-77 | 74-77 | 73-76 |
| BELOW AV | 79-84 | 77-82 | 79-84 | 78-83 | 78-83 | 77-84 |
| POOR | 85+ | 83+ | 85+ | 84+ | 84+ | 84+ |

# FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140257122 A
- US 20080004904 A
- US 20080157980 A

**Non-patent literature cited in the description**

- Recommendations for the Standardization and Interpretation of the Electrocardiogram Part VI: Acute Ischemia/Infarction. *Circulation,* 2009, vol. 119, e262-e27